(19) **Europäisches Patentamt**
**European Patent Office**
**Office européen des brevets**

(11) **EP 4 397 266 B1**

(12) **EUROPEAN PATENT SPECIFICATION**

(45) Date of publication and mention
of the grant of the patent:
**08.07.2026 Bulletin 2026/28**

(21) Application number: **24150507.2**

(22) Date of filing: **05.01.2024**

(51) International Patent Classification (IPC):
***A61B 18/18*** (2006.01)

(52) Cooperative Patent Classification (CPC):
**A61B 18/1815;** A61B 2017/00061;
A61B 2018/00023; A61B 2018/00577;
A61B 2018/00642; A61B 2018/0066;
A61B 2018/00702; A61B 2018/00785;
A61B 2018/00791; A61B 2018/00797

(54) **SYSTEM FOR OPTIC-BASED ABLATION ZONE SIMULATION**

SYSTEM ZUR OPTISCHEN ABLATIONSZONENSIMULATION

SYSTÈME DE SIMULATION DE ZONE D'ABLATION À BASE OPTIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **06.01.2023 US 202363437417 P
31.10.2023 US 202318498326**

(43) Date of publication of application:
**10.07.2024 Bulletin 2024/28**

(73) Proprietor: **Covidien LP
Mansfield, MA 02048 (US)**

(72) Inventors:
• **FRUSHOUR, Scott E. M.
Lafayette (CO), 80026 (US)**
• **KOMP, John W.
Lafayette (CO), 80026 (US)**
• **DICKHANS, William J.
Lafayette (CO), 80026 (US)**

(74) Representative: **Zimmermann & Partner
Patentanwälte mbB
Josephspitalstr. 15
80331 München (DE)**

(56) References cited:
**CN-A- 109 984 734     US-A1- 2021 361 353**

**Description**

FIELD

The present disclosure relates to systems, methods, and devices for simulating ablation zones in a microwave ablation treatment procedure using optic-based temperature measurements.

BACKGROUND

When planning a treatment procedure, clinicians often rely on patient data including X-ray data, computed tomography (CT) scan data, magnetic resonance imaging (MRI) data, or other imaging data that allows the clinician to view the internal anatomy of a patient. The clinician utilizes the patient data to identify targets of interest and to develop strategies for accessing the targets of interest for a treatment procedure such as a microwave ablation treatment procedure.

Microwave antennas generate heat in the body to provide hyperthermic temperatures to destroy tissues of interest. A challenge with radiated fields that are emitted from a highfrequency antenna is that it is difficult to measure the temperature profile induced in the tissue resulting from exposure to the radiated field, thus complicating the ability to determine the zone of ablation during the application of energy to the tissue.

Existing imaging modalities introduce unique challenges when monitoring the progression of an ablation zone during an ablation procedure. Ultrasound imaging displays "bubbles" or ground glass opacity as energy is delivered, making it difficult to determine the edge of the actual ablation zone during progression of the application of energy. CT imaging provides "near-time" imaging that reflects the ablation zone size at the time that the last scan was captured, however, does not reflect ablation growth as it occurs in real time.

Additionally, predicting the expected final ablation zone characteristics (e.g., dimensions, shape, etc. upon completion of the application of energy) and the progression of the actual ablation characteristics toward the final ablation zone characteristics is difficult as one standard data set used to predict the final ablation zone characteristics may not be generic to multiple patients and/or multiple targets and the clinician is unable to visualize the actual progression of the ablation zone in real time relative to the final ablation zone characteristics.

US 2021361353 discloses a microwave ablation system for sensing temperature at different locations on a microwave antenna.

SUMMARY

The invention is defined in the appended claims.

Systems and methods for simulating ablation zones in a microwave ablation treatment procedure using optic-based temperature measurements are provided.

According to an aspect of the present disclosure, an ablation system includes an ablation device and a computing device. The ablation device includes a radiating antenna configured to apply microwave ablation energy to a target within a patient to ablate the target and a fiber including Bragg gratings formed along a length thereof and configured to reflect light therefrom. The computing device includes a processor and memory storing instructions which, when executed by the processor, cause the computing device to calculate temperature measurements based on the reflected light during application of microwave ablation energy to the target and extrapolate the temperature measurements calculated to generate an ablation volume in real time.

In an aspect, the system may further include a display operably coupled to the computing device and the computing device may be configured to cause the display to display a progression of the ablation volume in real time. Additionally, or alternatively, the computing device may be configured to cause the display to overlay the ablation volume onto an image of the target within the patient. Additionally, or alternatively, the computing device may be configured to calculate a total expected ablation zone, cause the display to display the total expected ablation zone onto an image of the target within the patient, and cause the display to display the progression of the ablation volume within the display of the total expected ablation zone.

In an aspect, the Bragg gratings may be etched into the fiber.

In an aspect, the ablation device may be a flexible microwave ablation catheter configured to be navigated to the target through a patient's luminal network.

In an aspect, the ablation device may be a rigid microwave ablation device configured to be percutaneously inserted through tissue to access the target.

In an aspect, the computing device may be configured to generate the ablation volume based on the extrapolated temperature measurements, a total amount of microwave ablation energy applied, and a duration of energy application.

In an aspect, the computing device may be configured to determine an expected ablation zone geometry to be formed by the ablation device based on a model of the ablation device, and extrapolate the temperature measurements based on the expected ablation zone geometry determined. Additionally, or alternatively, the expected ablation zone geometry may be substantially spherically shaped and the computing device may be configured to extrapolate the temperature measurements based on the substantially spherical shape.

In accordance with another aspect of the disclosure, an ablation system includes a computing device. The computing device includes a processor and memory storing instructions which, when executed by the processor, cause the computing device to receive reflected light from fiber Bragg gratings of an ablation device, calculate temperature measurements based on the reflected light during application of microwave ablation energy by the ablation device to a target within a patient, and extrapolate the temperature measurements calculated to generate an ablation volume in real time. Additionally, the computing device is configured to cause a display device to display an image of the target, and cause the display device to overlay a progression of the ablation volume onto the image of the target.

In an aspect, the image of the target may be a three dimensional rendering of a portion of the patient generated from at least one of DICOM images or fluoroscopic images.

In an aspect, the computing device may be configured to calculate a total expected ablation zone, cause the display to display the total expected ablation zone onto an image of the target within the patient, and cause the display to display the progression of the ablation volume within the display of the total expected ablation zone.

In an aspect, the computing device may be configured to generate the ablation volume based on the extrapolated temperature measurements, a total amount of microwave ablation energy applied, and a duration of energy application.

In accordance with another aspect of the disclosure, a method for optic-based ablation zone simulation includes receiving reflected light from fiber Bragg gratings of an ablation device, calculating temperature measurements based on the reflected light during application of microwave ablation energy by the ablation device to a target within a patient, and extrapolating the temperature measurements calculated to generate an ablation volume in real time. The method further includes causing a display device to display an image of the target, and causing the display device to overlay a progression of the ablation volume onto the image of the target.

In an aspect, causing the display device to display the image of the target includes causing the display device to display a three dimensional rendering of a portion of the patient generated from at least one of DICOM images or fluoroscopic images.

In an aspect, the method may further include calculating a total expected ablation zone based on one or more of a type of ablation device, a total power setting, and a total duration of application of microwave energy. Additionally, or alternatively, the method may further include causing the display to display the total expected ablation zone calculated onto an image of the target within the patient. Additionally, or alternatively, the method may further include causing the display to display the progression of the ablation volume within the display of the total expected ablation zone calculated.

In an aspect, extrapolating the temperature measurements calculated to generate the ablation volume in real time may include generating the ablation volume based on the extrapolated temperature measurements, a total amount of microwave ablation energy applied, and a duration of energy application.

Any of the above aspects and embodiments of the present disclosure may be combined without departing from the scope of the present disclosure.

## BRIEF DESCRIPTION OF THE DRAWINGS

Objects and features of the presently disclosed system and method will become apparent to those of ordinary skill in the art when descriptions of various embodiments thereof are read with reference to the accompanying drawings, of which:

FIG. 1 is a schematic diagram of a microwave ablation procedure system in accordance with an illustrative aspect of the present disclosure;

FIG. 2 is a schematic diagram of a computing device which forms part of the microwave ablation procedure system of FIG. 1 in accordance with an aspect of the present disclosure;

FIG. 3A is a cross-sectional view of a distal portion of a flexible microwave ablation catheter in accordance with an aspect of the present disclosure;

FIG. 3B is a cross-sectional view of a rigid microwave ablation device in accordance with an aspect of the present disclosure;

FIG. 4 is a graph illustrating a temperature curve of measured temperature values and extrapolated temperature values of an ablation device in accordance with an aspect of the present disclosure;

FIG. 5 is an illustration of a volume of temperature gradients of measured temperature values and extrapolated temperature values of an ablation zone in accordance with an aspect of the present disclosure;

FIG. 6 is an example user interface displayable on a display of the microwave ablation procedure system of FIG. 1 in accordance with an aspect of the present disclosure; and

FIG. 7 is a flowchart illustrating a method for optic-based ablation zone simulation in accordance with an aspect of the present disclosure.

## DETAILED DESCRIPTION

The present disclosure provides a system and method for simulating and displaying ablation zones in real time in a microwave ablation treatment procedure using optic-based temperature measurements. In particular, the present disclosure provides an ablation device including fiber Bragg gratings for non-contact inferential sensing of various parameters including temperature, strain, pressure, torque and more. The gratings are embedded via laser etching inside a glass fiber with an explicitly known grating profile associated with the Bragg wavelength or frequency. Exposure to temperature and/or strain causes a broad-spectral pulse of light to reflect off the Bragg grating and shift from the center frequency either up or down in frequency at a known coefficient related to the parameter of interest.

Incorporating fiber Bragg gratings into a microwave ablation device enables real-time parameter sensing, for example real-time temperature sensing, which can be utilized to inform the treating physician of the dimensions of ablation volume being generated in real time as the application of ablation energy progresses.

Although the present disclosure will be described in terms of specific illustrative embodiments, it will be readily apparent to those skilled in the art that various modifications, rearrangements, and substitutions may be made without departing from the scope of the present disclosure. The scope of the present disclosure is defined by the claims appended hereto.

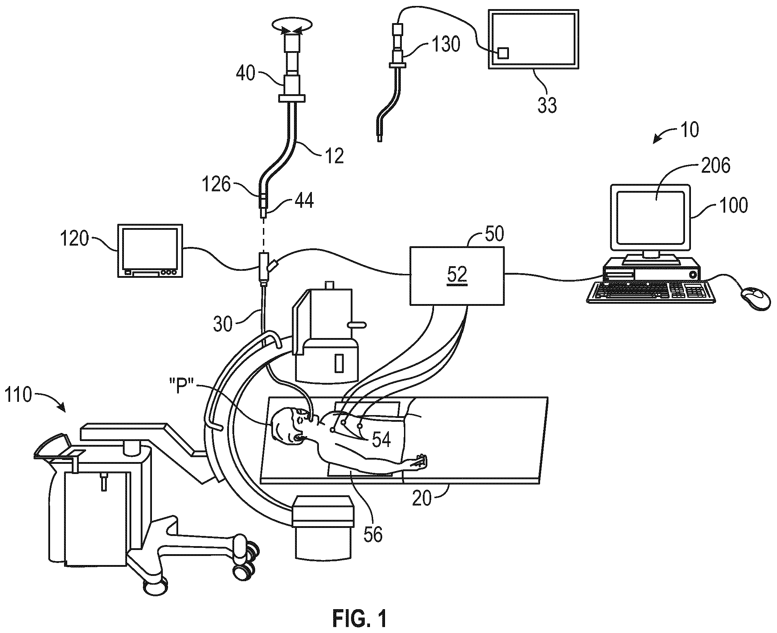

Fig. 1 depicts an Electromagnetic Navigation (EMN) system 10 configured for reviewing CT image data to identify one or more targets, planning a pathway to an identified target (planning phase), navigating a catheter 12 (e.g., an extended working channel) of a catheter guide assembly 40 to a target (navigation phase) via a user interface, and confirming placement of the catheter 12 relative to the target. One such EMN system is the ELECTROMAGNETIC NAVIGATION BRONCHOSCOPY® system currently sold by Medtronic plc. The target may be tissue of interest identified by review of the CT image data during the planning phase. Following navigation, a medical instrument, such as a biopsy tool, ablation tool (e.g., ablation device 130), or other tool, may be inserted into the catheter 12 to obtain a tissue sample (or perform any treatment) from the tissue located at, or proximate to, the target. Although CT image data is described, any form of imaging data by any imaging device may be utilized prior to, or during, a procedure.

As shown in Fig. 1, catheter 12 is part of a catheter guide assembly 40. In practice, the catheter 12 is inserted into bronchoscope 30 for access to a luminal network of patient "P." Specifically, catheter 12 of catheter guide assembly 40 may be inserted into a working channel of bronchoscope 30 for navigation through a patient's luminal network. A distal portion of the catheter 12 includes a sensor 44. The position and orientation of the sensor 44 relative to the reference coordinate system, and thus the distal portion of the catheter 12, within an electromagnetic field can be derived.

EMN system 10 generally includes an operating table 20 configured to support a patient "P;" a bronchoscope 30 configured for insertion through patient's "P's" mouth into patient's "P's" airways; monitoring equipment 120 coupled to bronchoscope 30 (e.g., a video display, for displaying the video images received from the video imaging system of bronchoscope 30); a tracking system 50 including a tracking module 52, a plurality of reference sensors 54 and a transmitter mat 56; and a computing device 100 including software and/or hardware used to facilitate identification of a target, pathway planning to the target, navigation of a medical instrument to the target, and confirmation of placement of an catheter 12, or a suitable device therethrough, relative to the target.

A fluoroscopic imaging device 110 capable of acquiring fluoroscopic or x-ray images or video of patient "P" is also included in this particular aspect of system 10. The fluoroscopic data (e.g., images, series of images, or video) captured by the fluoroscopic imaging device 110 may be stored within the fluoroscopic imaging device 110 or transmitted to computing device 100 for storage, processing, and display. Additionally, the fluoroscopic imaging device 110 may move relative to patient "P" so that images may be acquired from different angles or perspectives relative to patient "P" to create a fluoroscopic video from a fluoroscopic sweep. The pose of fluoroscopic imaging device 110 relative to patient "P" and while capturing the images may be estimated via markers incorporated with the transmitter mat 56. The markers are positioned under patient "P", between patient "P" and operating table 20 and between patient "P" and a radiation source or a sensing unit of fluoroscopic imaging device 110. The markers incorporated with the transmitter mat 56 may be two separate elements which may be coupled in a fixed manner or alternatively may be manufactured as a single unit. Fluoroscopic imaging device 110 may include a single imaging device or more than one imaging device. In embodiments including multiple imaging devices, each imaging device may be a different type of imaging device or the same type.

Computing device 100 may be any suitable computing device including a processor and storage medium, wherein the processor is capable of executing instructions stored on the storage medium. The computing device 100 may further include a database configured to store patient data, CT data sets including CT images, fluoroscopic data sets including fluoroscopic images and video, navigation plans, and any other such data. Although not explicitly illustrated, the computing device 100 may include inputs, or may otherwise be configured to receive, CT data sets, fluoroscopic images/video and other data described herein. Additionally, computing device 100 includes a display (e.g., display 206) configured to display graphical user interfaces.

With respect to the planning phase, computing device 100 utilizes previously acquired CT image data for generating and viewing a three-dimensional model or rendering of patient "P's" airways, enables the identification of a target on the three-dimensional model (automatically, semiautomatically, or manually), and allows for determining a pathway through patient "P's" airways to tissue located at and around the target. More specifically, CT images acquired from previous CT scans are processed and assembled into a three-dimensional CT volume, which is then utilized to generate a three-dimensional model of patient "P's" airways. The three-dimensional model may be displayed on a display 206 associated with computing device 100, or in any other suitable fashion. Using computing device 100, various views of the three-dimensional model or enhanced two-dimensional images generated from the three-dimensional model are presented. The enhanced two-dimensional images may possess some three-dimensional capabilities because they are generated from three-dimensional data. The three-dimensional model may be manipulated to facilitate identification of target on the three-dimensional model or two-dimensional images, and selection of a suitable pathway through patient "P's" airways to access tissue located at the target can be made. Once selected, the pathway plan, three-dimensional model, and images derived therefrom, can be saved and exported to a navigation system for use during the navigation phase(s). One such planning software is the ILLUMISITE® planning suite currently sold by Medtronic plc.

With respect to the navigation phase, a six degrees-of-freedom electromagnetic tracking system 50, or other suitable positioning measuring system, is utilized for performing registration of the images and the pathway for navigation, although other configurations are also contemplated. Tracking system 50 includes a tracking module 52, a plurality of reference sensors 54, and a transmitter mat 56 (including markers if applicable). Tracking system 50 is configured for use with a sensor 44 of catheter guide assembly 40 to track the electromagnetic position thereof within an electromagnetic coordinate system.

Transmitter mat 56 is positioned beneath patient "P." Transmitter mat 56 generates an electromagnetic field around at least a portion of patient "P" within which the position of a plurality of reference sensors 54 and the sensor 44 can be determined with use of a tracking module 52. A second electromagnetic sensor 126 may also be incorporated into the end of the catheter 12. The second electromagnetic sensor 126 may be a five degree-of-freedom sensor or a six degree-of-freedom sensor. One or more of reference sensors 54 are attached to the chest of patient "P." The six degrees of freedom coordinates of reference sensors 54 are sent to computing device 100 (which includes the appropriate software) where they are used to calculate a patient coordinate frame of reference. Registration, as detailed below, is generally performed to coordinate locations of the three dimensional model and two dimensional images from the planning phase with patient's "P's" airways as observed through the bronchoscope 30, and allow for the navigation phase to be undertaken with precise knowledge of the location of the sensor 44, even in portions of the airway where the bronchoscope 30 cannot reach.

Registration of patient's "P's" location on the transmitter mat 56 is performed by moving sensor 44 through the airways of patient's "P." More specifically, data pertaining to locations of sensor 44, while catheter 12 is moving through the airways, is recorded using transmitter mat 56, reference sensors 54, and tracking module 52. A shape resulting from this location data is compared to an interior geometry of passages of the three dimensional model generated in the planning phase, and a location correlation between the shape and the three dimensional model based on the comparison is determined, e.g., utilizing the software on computing device 100. In addition, the software identifies non-tissue space (e.g., air filled cavities) in the three dimensional model. The software aligns, or registers, an image representing a location of sensor 44 with the three dimensional model and two dimensional images generated from the three dimension model, which are based on the recorded location data and an assumption that sensor 44 remains located in non-tissue space in patient's "P's" airways. Alternatively, a manual registration technique may be employed by navigating the bronchoscope 30 with the sensor 44 to pre-specified locations in the lungs of patient "P", and manually correlating the images from the bronchoscope to the model data of the three dimensional model.

Though described herein with respect to EMN systems using EM sensors, the instant disclosure is not so limited and may be used in conjunction with flexible sensor, ultrasonic sensors, or without sensors. Additionally, the methods described herein may be used in conjunction with robotic systems such that robotic actuators drive the catheter 12, catheter guide assembly 40 components, or bronchoscope 30 proximate the target.

Following registration of patient "P" to the image data and pathway plan, a user interface is displayed in the navigation software which sets forth the pathway that the clinician is to follow to reach the target.

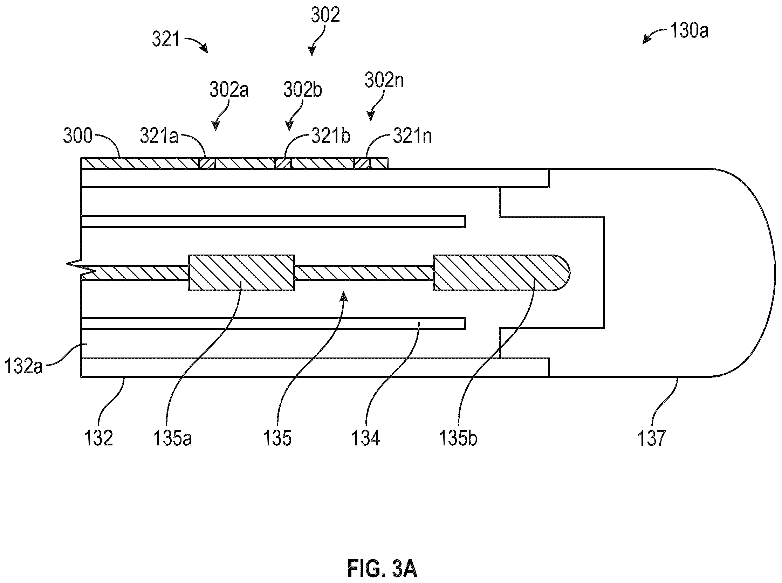

Once catheter 12 has been successfully navigated proximate the target as depicted on the user interface, the catheter 12 is in place as a guide channel for guiding medical instruments including without limitation, optical systems, ultrasound probes, marker placement tools, biopsy tools, ablation tools (i.e., microwave ablation devices), laser probes, cryogenic probes, sensor probes, and aspirating needles to the target. In an aspect, ablation device 130 is a flexible microwave ablation catheter 130a (FIG. 3A) which is guided through catheter 12 for placement relative to a target and ablation of the target.

Prior to inserting the medical device through the catheter 12, a local registration process may be performed for each target to reduce the CT-to-body divergence. In a capture phase of the local registration process, a sequence of fluoroscopic images may be captured and acquired via fluoroscopic imaging device 110, optionally by a user and according to directions displayed via computing device 100. A fluoroscopic 3D reconstruction may be then generated via computing device 100. The generation of the fluoroscopic 3D reconstruction is based on the sequence of fluoroscopic images and the projections of structure of markers incorporated with transmitter mat 56 on the sequence of images. One or more slices of the 3D reconstruction may be then generated based on the pre-operative CT scan and via computing device 100. The one or more slices of the 3D reconstruction and the fluoroscopic 3D reconstruction may be then displayed to the user on a display via computing device 100, optionally simultaneously. The slices of 3D reconstruction may be presented on the user interface in a scrollable format where the user is able to scroll through the slices in series. Additionally, or alternatively, other imaging modalities such as CT or cone beam CT may be utilized.

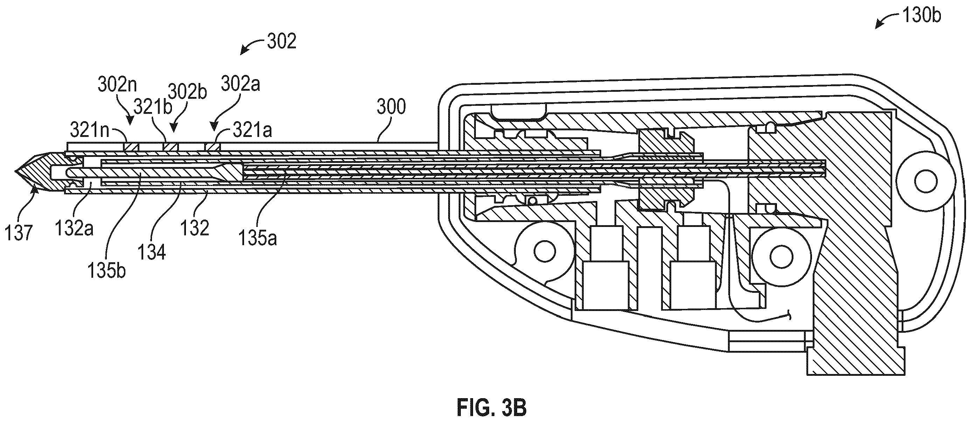

Ablation device 130 is a surgical instrument having a microwave ablation antenna that is used to ablate tissue, such as a lesion or tumor, by using electromagnetic radiation or microwave energy to heat tissue in order to denature or kill cancerous cells. Ablation device 130 may be a flexible microwave ablation catheter 130a (FIG. 3A) configured to be navigated to a target via a patient's luminal network through a catheter 12 that has been navigated to the target. Alternatively, ablation device 130 may be a rigid microwave ablation device 130b (FIG. 3B) configured to be percutaneously inserted through tissue to access a target within a patient. Ablation device 130 is configured to connect to microwave generator 33 (FIG. 1) which generates and controls the application of microwave energy through the ablation device 130. Microwave generator 33 may be a component of computing device 100 or may be a separate stand-alone component. Ablation device 130 is described in further detail below with respect to FIGS. 3A and 3B.

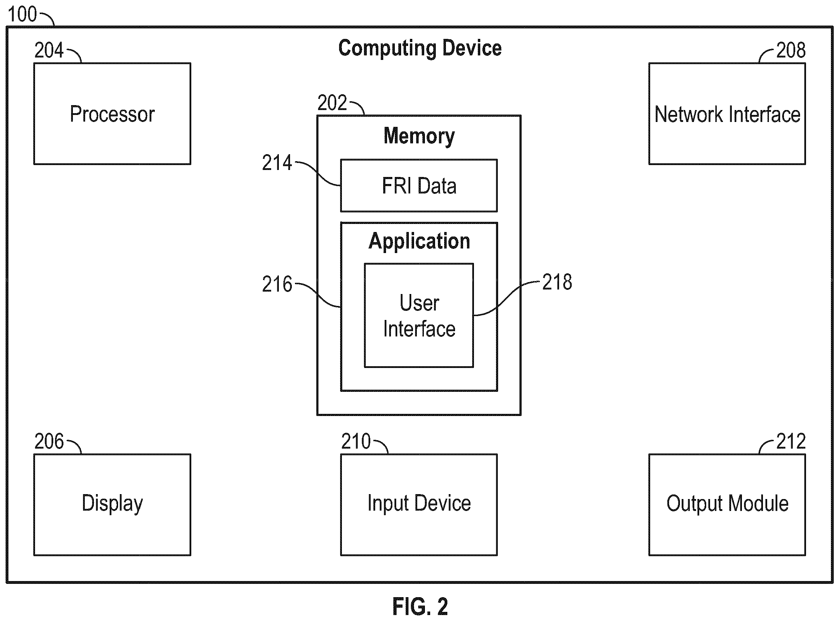

FIG. 2 illustrates a system diagram of computing device 100. Computing device 100 may include memory 202, processor 204, display 206, network interface 208, input device 210, and/or output module 212. Memory 202 includes any non-transitory computer-readable storage media for storing data and/or software that is executable by processor 204 and which controls the operation of computing device 100. In an embodiment, memory 202 may include one or more solid-state storage devices such as flash memory chips. Alternatively or in addition to the one or more solid-state storage devices, memory 202 may include one or more mass storage devices connected to the processor 204 through a mass storage controller (not shown) and a communications bus (not shown). Although the description of computer-readable media contained herein refers to a solid-state storage, it should be appreciated by those skilled in the art that computer-readable storage media can be any available media that can be accessed by the processor 204. That is, computer readable storage media includes non-transitory, volatile and non-volatile, removable and non-removable media implemented in any method or technology for storage of information such as computer-readable instructions, data structures, program modules, or other data. For example, computer-readable storage media includes RAM, ROM, EPROM, EEPROM, flash memory or other solid state memory technology, CD-ROM, DVD, Blu-Ray or other optical storage, magnetic cassettes, magnetic tape, magnetic disk storage or other magnetic storage devices, or any other medium which can be used to store the desired information and which can be accessed by computing device 100.

Memory 202 may store application 216 and/or functional respiratory imaging data 214 of one or more patients. Application 216 may, when executed by processor 204, cause display 206 to present user interfaces. Processor 204 may be a general-purpose processor, a specialized graphics processing unit (GPU) configured to perform specific graphics processing tasks while freeing up the general-purpose processor to perform other tasks, and/or any number or combination of such processors. Display 206 may be touch sensitive and/or voice activated, enabling display 206 to serve as both an input and output device. Alternatively, a keyboard (not shown), mouse (not shown), or other data input devices may be employed. Network interface 208 may be configured to connect to a network such as a local area network (LAN) consisting of a wired network and/or a wireless network, a wide area network (WAN), a wireless mobile network, a Bluetooth network, and/or the internet. For example, computing device 100 may receive functional respiratory imaging data, DICOM imaging data, computed tomographic (CT) image data, or other imaging data, of a patient from an imaging workstation 150 and/or a server, for example, a hospital server, internet server, or other similar servers, for use during surgical ablation planning. Patient functional respiratory imaging data may also be provided to computing device 100 via a removable memory 202. Computing device 100 may receive updates to its software, for example, application 216, via network interface 208. Computing device 100 may also display notifications on display 206 that a software update is available.

Input device 210 may be any device by means of which a user may interact with computing device 100, such as, for example, a mouse, keyboard, foot pedal, touch screen, and/or voice interface. Output module 212 may include any connectivity port or bus, such as, for example, parallel ports, serial ports, universal serial busses (USB), or any other similar connectivity port known to those skilled in the art.

Application 216 may be one or more software programs stored in memory 202 and executed by processor 204 of computing device 100. During a planning phase, application 216 guides a clinician through a series of steps to identify a target, size the target, size a treatment zone, and/or determine an access route to the target for later use during the procedure phase. In some embodiments, application 216 is loaded on computing devices in an operating room or other facility where surgical procedures are performed, and is used as a plan or map to guide a clinician performing a surgical procedure, but without any feedback from ablation device 130 used in the procedure to indicate where ablation device 130 is located in relation to the plan. In other embodiments, system 10 provides computing device 100 with data regarding the location of ablation device 130 within the body of the patient, such as by EM tracking, which application 216 may then use to indicate on the plan where ablation device 130 is located.

Application 216 may be installed directly on computing device 100, or may be installed on another computer, for example, a central server, and opened on computing device 100 via network interface 208. Application 216 may run natively on computing device 100, as a web-based application, or any other format known to those skilled in the art. In some embodiments, application 216 will be a single software program having all of the features and functionality described in the present disclosure. In other embodiments, application 216 may be two or more distinct software programs providing various parts of these features and functionality. For example, application 216 may include one software program for use during the planning phase, and a second software program for use during the procedure phase of the microwave ablation treatment. In such instances, the various software programs forming part of application 216 may be enabled to communicate with each other and/or import and export various settings and parameters relating to the microwave ablation treatment and/or the patient to share information. For example, a treatment plan and any of its components generated by one software program during the planning phase may be stored and exported to be used by a second software program during the procedure phase.

Application 216 communicates with a user interface 218 that generates a user interface for presenting visual interactive features to a clinician, for example, on display 206 and for receiving clinician input, for example, via a user input device. For example, user interface 218 may generate a graphical user interface (GUI) and output the GUI to display 206 for viewing by a clinician.

Computing device 100 is linked to display 120, thus enabling computing device 100 to control the output on display 120 along with the output on display 206. Computing device 100 may control display 120 to display output which is the same as or similar to the output displayed on display 206. For example, the output on display 206 may be mirrored on display 120. Alternatively, computing device 100 may control display 120 to display different output from that displayed on display 206. For example, display 120 may be controlled to display guidance images and information during the microwave ablation procedure, while display 206 is controlled to display other output, such as configuration or status information.

FIG. 3A illustrates a distal portion of a flexible microwave ablation catheter 130a and FIG. 3B illustrates a rigid microwave ablation device 130b (referred to collectively as ablation device 130). The ablation device 130 includes a distal tip 137 which may be blunt or may include a tapered trocar tip for percutaneous insertion through tissue. The ablation device 130 includes a radiating antenna 135 which includes a proximal radiating section 135a and a distal radiating section 135b, which are configured to emit microwave energy into the tissue surrounding the radiating antenna 135. A cooling tube 134 is positioned relative to the radiating antenna 135 for passage of a cooling fluid therethrough to cool the radiating antenna 135 during application of microwave energy. An outer jacket 132 defines a lumen 132a and surrounds both of the cooling tube 134 and the radiating antenna 135. The cooling tube 134 may be positioned adjacent and alongside the radiating antenna 135 within the lumen 132a of the outer jacket 132 or the radiating antenna 135 may be positioned within the cooling tube 134. In percutaneous applications, the rigid microwave ablation device 130b includes an outer jacket 132 formed of a rigid material so as to maintain it's shape during percutaneous insertion of the rigid microwave ablation device 130b through tissue to a target. Alternatively, in lung navigation applications, the flexible microwave ablation catheter 130a may include an outer jacket 132 which is formed of a flexible or semi-rigid material capable of being navigated through a catheter (e.g., catheter 12) to a target.

The ablation device 130 includes a fiber 300 which may be coupled to the outer jacket 132. In aspects, the fiber 300 is embedded into the outer jacket 132. Alternatively, the fiber 300 may be positioned in the lumen 132a of the outer jacket 132 or on an outer surface of the outer jacket 132. In aspects, the fiber 300 may be retrofitted to an ablation device 130 by securing the fiber 300 to the outer jacket 132 via heat-shrink or other mediums. A proximal end of the fiber 300 may be incorporated into a connector of the ablation device 130 which connects the ablation device 130 to a microwave generator (e.g., microwave generator 33), or alternatively, a separate independent connector may be utilized to connect the proximal end of the fiber 300 to the microwave generator, computing device 100, or another component of system 10.

Fiber 300 includes a plurality of Bragg gratings 302a, 302b, ...302n (referred to collectively as Bragg gratings 302) etched into the fiber 300. The Bragg gratings 302 reflect a narrow wavelength range called the Bragg wavelength. Each Bragg grating 302 includes periodic modulations in the core of the fiber 300 with spacing between each modulation. This changes the refractive index of the fiber 300 so that a single wavelength is reflected, while the rest of the light is transmitted down the fiber 300. The spacing between modulations changes when a Bragg grating 302 is subjected to a change in temperature. This changes the refractive index of the Bragg grating 302 and causes the Bragg wavelength to shift. Embodiments of the present disclosure use the shift in the Bragg wavelength to determine a temperature. The Bragg gratings 302 may be integrated at points of interest along the length of the ablation device 130. For example, in aspects, the Bragg gratings 302 are spaced 10-50 mm on the proximal length of the ablation device 130 and spaced between 1-5 mm at the distal portion of the ablation device 130 where electromagnetic radiation and subsequent temperature change is likely to occur.

The Bragg gratings 302 include a plurality of reflection points 321a, 321b, ...321n (referred to collectively as reflection points 321) written into the fiber 300 at periodic spacing "A." In some embodiments, the Bragg gratings 302 may be written into the fiber 300 using high intensity pulses from a laser (e.g., argon fluoride excimer laser with a phase mask). As the fiber 300 undergoes mechanical strain (e.g., a change in length) due to temperature and pressure changes, the spacing A is modified due to stretching or contraction of the fiber 300. The effects of changes in temperature on the fiber 300 is quantified by the computing device 100 by measuring the wavelength shift in light reflected by the reflection points 321 based on the following equation:

$$\frac{\Delta\lambda}{\lambda_0} = k * \varepsilon + \alpha_\delta * \Delta T \tag{1}$$

In equation (1), $\Delta\lambda$ is the wavelength shift, $\lambda 0$ is the base wavelength, k is a gauge factor, which is a difference between 1 and a photo-elastic coefficient, $\rho$, $\varepsilon$ is strain, $\Delta T$ is a temperature change, and $\alpha\delta$ is a change of the refraction index.

In this manner, fiber 300 is configured to transmit at least one wavelength of light and the Bragg gratings 302 are configured to reflect at least one wavelength of light. Thus, the light transmissive properties, namely transmittance, of the distal portion of the fiber 300 corresponds to a set of physical parameters of the ablation device 130 or the environment in which it is positioned (e.g., temperature of tissue). In particular, changes in temperature and pressure affect (e.g., stretch or contract) the distal portion of the optical fiber 300, which, in turn, modifies the spacing between the Bragg gratings 302. The changes in spacing between the Bragg gratings 302 changes the wavelength of the light reflected back through the fiber 300. The change in the wavelength is then used by the computing device 100 to determine the change in temperatures at the locations of the Bragg gratings 302.

The computing device 100 includes a fiber grating demodulator, which demodulates the reflected light transmitted through fiber 300 using a demodulation technique to obtain the changes in wavelength. Demodulation techniques include wavelength division multiplexing (WDM), optical time domain reflectometry (OTDM), optical frequency domain reflectometry (OFDM), and code correlation techniques that incorporate aspects of OTDM and OFDM. According to the OTDR technique, a narrow light pulse is generated by a light source and is transmitted through the optical fiber 300 to the fiber Bragg gratings 302. The reflected or backscattered light is analyzed to determine multiple temperatures. The locations corresponding to each of the temperatures may be determined by monitoring the time it takes the reflected or backscattered light to return to the photodetector.

The temperature measurements sensed by computing device 100 may be displayed and/or may be used to control aspects of the system 10, for example, to control output of the microwave generator 33. In embodiments, the data is extrapolated during application of microwave ablation energy to simulate the volumetric geometry of the ablation zone as the ablation procedure progresses. In particular, the computing device 100 observes a gradient of temperature or strain shift by the grating wavelength shifts in the fiber 300 when the radiating antenna 135 of the ablation device 130 is emitting microwave energy. This gradient should be at a peak at a location nearest to the radiating antenna 135 and is expected to fall off to a minimum at a point furthest away from the radiating antenna 135. At some distance from the radiating antenna 135, where the electromagnetic field has dropped off substantially (e.g., above or below a predetermined threshold), the temperature will approach an asymptote that matches the environmental temperature surrounding the radiating antenna 135 (e.g., the assumed temperature of the human body of 37 degrees C).

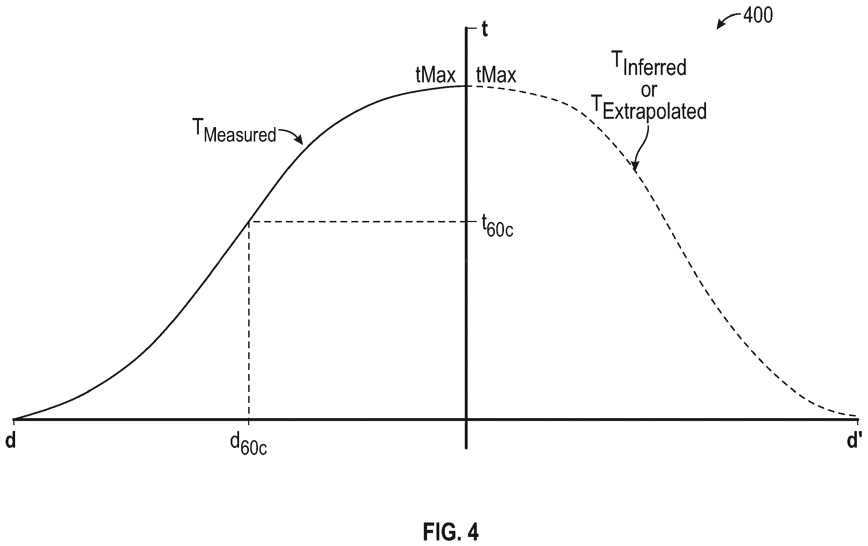

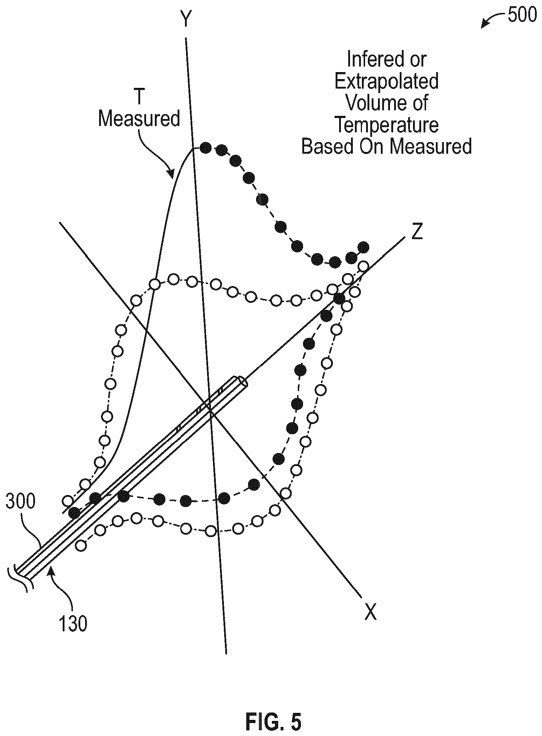

With reference to FIGS. 4 and 5, utilizing the temperature curve 400 of measured temperature values and extrapolated or inferred temperature values, computing device 100 may sweep the temperature curve 400 around all three axes (X, Y, Z) to create a volume of temperature gradients 500. A region of interest may be the point where 60 degrees C is measured and the range of tissue below 60 degrees C is measured as this is where cell death should not be expected. Parameters to a given procedure (e.g., ablation zone dimensions, ablation zone edges, etc.) may be identified based on temperature values alone, total energy deposited (time x power), and/or temperature dwell periods to identify a region of likely necrosis. The total final expected ablation zone (derived from power to be delivered x time to be delivered, for example) may be projected and compared to the actual ablation zone dimensions in real time as the thermal field grows during the application of microwave energy. In this manner, a clinician may visualize the progression of the ablation zone relative to the desired final ablation zone to be expected upon the full application of energy for the full time allotted, thus providing intraprocedural guidance to how the treatment is developing and progressing in real time.

Utilizing the example shown in FIGS. 4 and 5, the radiated field may be assumed to be perfectly spherical or substantially spherical (e.g., an ablation zone with a shape approaching a sphere but not necessarily perfectly spherical) which is appropriate for certain models of ablation devices 130. For other ablation devices configured to generate non-spherical ablation zones (e.g., ovoid, elongated, oblong, etc.), K parameters may be incorporated into the volumetric calculations and compared with pre-operative known in-vivo measurements to tune the intraoperative expected volume and measured volumes to match each other. Such K parameters can be known and stored within the memory of the computing device 100 or may be based on calibration parameters stored within the ablation device 130.

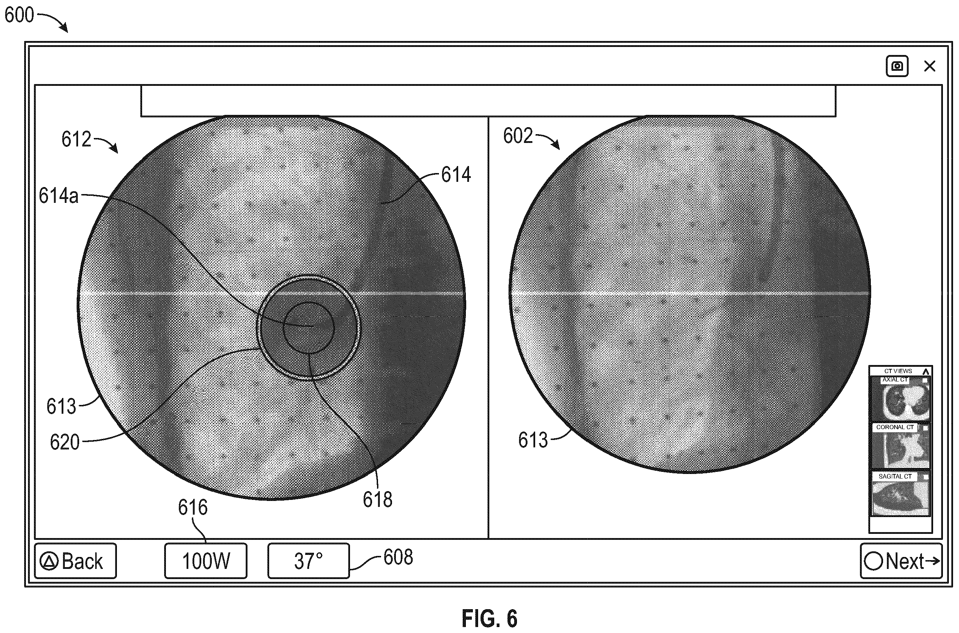

FIG. 6 illustrates an example user interface 600 displayable on one or both of display 120 and display 206 during an ablation procedure. During an ablation procedure, the clinician navigates ablation device 130 (displayed as ablation device 614 on the user interface 600) along a pathway to the target (e.g., utilizing an imaging device such as, for example, a fluoroscopic, CT, ultrasound, or hybrid three dimensional fluoro-CT imaging device, to visualize placement of the ablation device 130 relative to the target). While ablation device 130 is navigated, application 216 tracks the location of ablation device 130 or a catheter through which the ablation device 130 is placed inside the patient's body and displays the tracked location of ablation device 130 or catheter overlayed onto imaging data of the patient (e.g., patient CT data, ultrasound imaging data, fluoroscopic imaging data, hybrid three dimensional fluoro-CT imaging data, etc.) on the user interface. In addition, the application 216 may project a vector displayable on the user interface 600 extending from the end of the ablation device 130 to give an indication to the clinician of the intersecting tissue along the trajectory of the ablation device 130. In this manner, the clinician can alter the approach to a lesion or tumor to optimize the placement with minimum of trauma or follow a planned pathway to the target.

User interface 600 includes a view 602 of the patient images 613 captured during the procedure or captured preoperatively. For example, view 602 may include a three dimensional rendering of the patient's lungs generated from DICOM data. User interface 600 also includes a view for displaying status messages relating to the ablation procedure, such as a power setting 616 of the ablation device 130 or generator 33, duration of the ablation and/or a time remaining until the ablation procedure is complete, progression of the ablation, and/or temperature feedback 608 from a temperature sensor. User interface 600 also displays the navigation view 612, which includes a representation 314 of ablation device 130 as well as a shadow indicator 614a representing the portion of ablation device 130 which lies below a displayed two dimensional or three dimensional imaging plane, a simulation of ablation growth 618 representing the progression of the growth of the ablation zone during application of energy overlayed onto the patient images 613, and a total predicted ablation zone 620 representing the area which will be ablated if the ablation procedure is allowed to run to completion. The navigation view 612 may include three dimensional renderings generated from DICOM image data, for example, a three dimensional rendering generated from a CT scan of the patient.

As described above, the computing device extrapolates the volumetric dimensions of the ablation zone from the fiber optic-based temperature measurements which is displayed as the simulation of ablation growth 618 over time. The simulation of ablation growth 618 may include a solid outer edge which increases in size based on the duration of energy application and/or the real time fiber optic-based temperature measurements, a jagged line outer edge which moves and increases in size based on the duration of energy application and/or the real time fiber optic-based temperature measurements, and/or a pulsing line outer edge which fades between appearing and disappearing and which increases in size based on the duration of energy application and/or the real time fiber optic-based temperature measurements. The appearing and disappearing pulsing line of the simulation of ablation growth 618 prevents the simulation of ablation growth 618 from blocking visibility of objects in the displayed patient images 613.

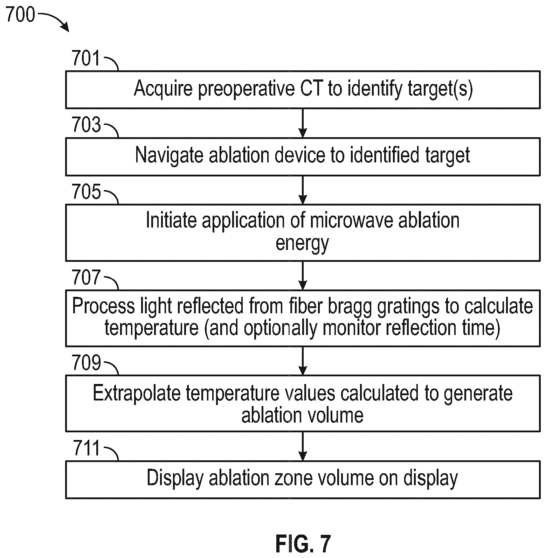

Turning to FIG. 7, a method for generating and displaying an ablation zone volume is illustrated and described as method 700. Method 700 is described as being executed by computing device 100, but some or all of the steps of method 700 may be implemented by one or more other components of the system 10, alone or in combination. Additionally, although method 700 is illustrated and described as including specific steps, and is described as being carried out in a particular order, it is understood that method 700 may include some or all of the steps described and may be carried out in any order not specifically described.

Method 700 begins at step 701 where computing device 100 acquires preoperative CT data of a patient's lungs to identify one or more targets requiring ablation. In step 703, an ablation device 130 is navigated to the target. For example, the ablation device 130 may be a flexible microwave ablation catheter 130a (FIG. 3A) which is navigated to the target through a patient's luminal airways. Alternatively, the ablation device 130 may be a rigid microwave ablation device 130b (FIG. 3B) which is percutaneously inserted through a patient's tissue to access the target.

In step 705, subsequent to final placement of the ablation device 130 relative to the target, the application of microwave energy to the ablation device 130 for ablating the target is initiated. In step 707, the computing device 100 processes the light reflected from fiber Bragg gratings 302 of the ablation device 130 to calculate a temperature at the fiber Bragg gratings 302, for example along a length of the ablation device 130. Step 707 may additionally include monitoring the amount of time it takes the reflected or backscattered light to return to a photodetector and determining the location of the temperature measurement along the length of the ablation device 130 corresponding to each reflected light signal based on the amount of time monitored.

In step 709, the computing device 100 extrapolates the temperature values calculated in step 707 to generate an ablation volume over the course of the duration of the ablation procedure. The ablation volume of step 709 may additionally be based on additional factors such as duration of energy application, total energy deposited, and/or temperature dwell periods. Step 709 may additionally include identifying a perimeter of an ablation zone based on one or more of the factors listed above. Step 709 is carried out by computing device throughout the course of the application of microwave ablation energy as the size of the ablation zone volume increases, in real time to show the growth of the ablation zone volume as the ablation procedure progresses.

In step 711, the ablation volume generated in step 709 is displayed on a display of computing device 100 for viewing by a clinician during the application of microwave ablation energy. The display of the generated ablation zone volume in step 711 is constantly updated in real time as the ablation zone increases in time. In this manner, the clinician is able to visualize a simulation of ablation zone growth as the procedure progresses. Step 711 may include overlaying the simulated ablation zone onto preoperative or intraoperative images of the patient. Additionally, the displayed simulation of the ablation zone volume may be displayed relative to an estimated total ablation zone, which represents the total ablation zone expected to be achieved upon completion of the ablation procedure, so that clinician can easily visualize the extent of the ablation zone and the remaining are expected to be ablated.

Although embodiments have been described in detail with reference to the accompanying drawings for the purpose of illustration and description, it is to be understood that the inventive processes and apparatus are not to be construed as limited thereby. It will be apparent to those of ordinary skill in the art that various modifications to the foregoing embodiments may be made without departing from the scope of the disclosure.

**Claims**

1.  An ablation system comprising:

    an ablation device (130) configured to deliver microwave ablation energy and comprising a fiber (300) including Bragg gratings; and
    a computing device (100) including a processor (204) and memory (202) storing instructions which, when executed by the processor, cause the computing device to:

        receive reflected light from the Bragg gratings (300, 302a ...302n) of the ablation device (130);
        calculate temperature measurements based on the reflected light during application of microwave ablation energy by the ablation device to a target within a patient;
        extrapolate the temperature measurements calculated to simulate an ablation volume in real time;

        cause a display device (120, 206) to display an image (613) of the target; and
        cause the display device to overlay a progression of the ablation volume onto the image of the target using a simulation of ablation growth (618) representing the progression of growth of the ablation volume.

2.  The ablation system of claim 1, the ablation device including:
    a radiating antenna (135) configured to apply microwave ablation energy to a target within a patient to ablate the target; wherein the Bragg gratings (302a, ...302n) are formed along a length of the fiber (300) and configured to reflect light therefrom.

3.  The ablation system of claim 2, wherein the Bragg gratings are etched into the fiber.

4.  The ablation system of claim 2 or 3, wherein the ablation device is a flexible microwave ablation catheter (130a) configured to be navigated to the target through a patient's luminal network.

5.  The ablation system of any of claims 2 to 4, wherein the ablation device is a rigid microwave ablation device (130b) configured to be percutaneously inserted through tissue to access the target.

6.  The ablation system of any of any of claims 2 to 5, wherein the computing device is configured to simulate the ablation volume based on the extrapolated temperature measurements, a total amount of microwave ablation energy applied, and a duration of energy application.

7.  The ablation system of any of claims 2 to 6, wherein the computing device is configured to:

        determine an expected ablation zone geometry (620) to be formed by the ablation device based on a model of the ablation device; and
        extrapolate the temperature measurements based on the expected ablation zone geometry determined.

8.  The ablation system of claim 7, wherein the expected ablation zone geometry is substantially spherically shaped, and the computing device is configured to extrapolate the temperature measurements based on the substantially spherical shape.

9.  The ablation system of claim 1, wherein the image of the target is a three dimensional rendering of a portion of the patient generated from at least one of DICOM images or fluoroscopic images.

10. The ablation system of claim 1 or claim 9, wherein the computing device is configured to:

calculate a total expected ablation zone;
cause the display to display the total expected ablation zone onto an image of the target within the patient; and
cause the display to display the progression of the ablation volume within the display of the total expected ablation zone.

11. The ablation system of any of claims 1, 9 or 10, wherein the computing device is configured to simulate the ablation volume based on the extrapolated temperature measurements, a total amount of microwave ablation energy applied, and a duration of energy application.

12. A method of operating a computing device in an ablation system, the computing device including a processor and memory storing instructions which, when executed by the processor, cause the computing device to perform the method of:

calculating temperature measurements based on reflected light from fiber Bragg gratings of an ablation device during application of microwave ablation energy by the ablation device to a target within a patient;
extrapolating the temperature measurements calculated to simulate an ablation volume in real time;
causing a display device to display an image of the target; and
causing the display device to overlay a progression of the ablation volume onto the image of the target, using a simulation of ablation growth (618) representing the progression of growth of the ablation zone.

13. The method of claim 12, wherein the image of the target is a three-dimensional rendering of a portion of the patient generated from at least one of DICOM images or fluoroscopic images.

14. The method of claim 12 or claim 13, wherein the computing device is further configured to perform the method of:

calculating a total expected ablation zone;
causing the display to display the total expected ablation zone onto an image of the target within the patient; and
causing the display to display the progression of the ablation volume within the display of the total expected ablation zone.

15. The method of any of claims 12 to 14, wherein the computing device is further configured to simulate the ablation volume based on the extrapolated temperature measurements, a total amount of microwave ablation energy applied, and a duration of energy application.

**Patentansprüche**

1. Ablationssystem, das Folgendes umfasst:

eine Ablationsvorrichtung (130), die dazu konfiguriert ist, Mikrowellenablationsenergie zu liefern, und die eine Faser (300) einschließlich Bragg-Gittern umfasst; und
eine Rechenvorrichtung (100), die einen Prozessor (204) und einen Speicher (202) beinhaltet, der Anweisungen speichert, die, wenn sie durch den Prozessor ausgeführt werden, die Rechenvorrichtung zu Folgendem veranlassen:

Empfangen von reflektiertem Licht von den Bragg-Gittern (300, 302a ...302n) der Ablationsvorrichtung (130);
Berechnen von Temperaturmessungen basierend auf dem reflektierten Licht während einer Anwendung von Mikrowellenablationsenergie durch die Ablationsvorrichtung auf ein Ziel innerhalb eines Patienten;
Extrapolieren der berechneten Temperaturmessungen, um ein Ablationsvolumen in Echtzeit zu simulieren;
Bewirken, dass eine Anzeigevorrichtung (120, 206) ein Bild (613) des Ziels anzeigt; und
Bewirken, dass die Anzeigevorrichtung eine Progression des Ablationsvolumens auf das Bild des Ziels unter Verwendung einer Simulation eines Ablationswachstums (618) überlagert, die die Progression eines Wachstums des Ablationsvolumens repräsentiert.

2. Ablationssystem nach Anspruch 1, wobei die Ablationsvorrichtung Folgendes beinhaltet:
eine Strahlungsantenne (135), die dazu konfiguriert ist, Mikrowellenablationsenergie auf ein Ziel innerhalb eines Patienten anzuwenden, um das Ziel zu abladieren; wobei die Bragg-Gitter (302a, ...302n) entlang einer Länge der Faser (300) gebildet und dazu konfiguriert sind, Licht von dieser zu reflektieren.

3. Ablationssystem nach Anspruch 2, wobei die Bragg-Gitter in die Faser geätzt sind.

4. Ablationssystem nach Anspruch 2 oder 3, wobei die Ablationsvorrichtung ein flexibler Mikrowellenablationskatheter (130a) ist, der dazu konfiguriert ist, durch ein Lumennetzwerk eines Patienten zu dem Ziel navigiert zu werden.

5. Ablationssystem nach einem der Ansprüche 2 bis 4, wobei die Ablationsvorrichtung eine starre Mikrowellenablationsvorrichtung (130b) ist, die dazu konfiguriert ist, perkutan durch Gewebe eingeführt zu werden, um Zugang zu dem Ziel zu erlangen.

6. Ablationssystem nach einem der Ansprüche 2 bis 5, wobei die Rechenvorrichtung dazu konfiguriert ist, das Ablationsvolumen basierend auf den extrapolierten Temperaturmessungen, einer Gesamtmenge an angewandter Mikrowellenablationsenergie und einer Dauer einer Energieanwendung zu simulieren.

7. Ablationssystem nach einem der Ansprüche 2 bis 6, wobei die Rechenvorrichtung ferner zu Folgendem konfiguriert ist:

     Bestimmen einer erwarteten Ablationszonengeometrie (620), die durch die Ablationsvorrichtung gebildet werden soll, basierend auf einem Modell der Ablationsvorrichtung; und Extrapolieren der Temperaturmessungen basierend auf der erwarteten Ablationszonengeometrie, die bestimmt wurde.

8. Ablationssystem nach Anspruch 7, wobei die erwartete Ablationszonengeometrie im Wesentlichen sphärisch geformt ist und die Rechenvorrichtung dazu konfiguriert ist, die Temperaturmessungen basierend auf der im Wesentlichen sphärischen Form zu extrapolieren.

9. Ablationssystem nach Anspruch 1, wobei das Bild des Ziels ein dreidimensionales Rendering eines Teils des Patienten ist, das aus DICOM-Bildern und/oder fluoroskopischen Bildern erzeugt wird.

10. Ablationssystem nach Anspruch 1 oder Anspruch 9, wobei die Rechenvorrichtung zu Folgendem konfiguriert ist:

     Berechnen einer gesamten erwarteten Ablationszone; Bewirken, dass die Anzeige die gesamte erwartete Ablationszone auf einem Bild des Ziels innerhalb des Patienten anzeigt; und Bewirken, dass die Anzeige die Progression des Ablationsvolumens innerhalb der Anzeige der gesamten erwarteten Ablationszone anzeigt.

11. Ablationssystem nach einem der Ansprüche 1, 9 oder 10, wobei die Rechenvorrichtung dazu konfiguriert ist, das Ablationsvolumen basierend auf den extrapolierten Temperaturmessungen, einer Gesamtmenge an angewandter Mikrowellenablationsenergie und einer Dauer einer Energieanwendung zu simulieren.

12. Verfahren zum Betreiben einer Rechenvorrichtung in einem Ablationssystem, wobei die Rechenvorrichtung einen Prozessor und einen Speicher beinhaltet, der Anweisungen speichert, die, wenn sie durch den Prozessor ausgeführt werden, die Rechenvorrichtung dazu veranlassen, das Verfahren aus Folgendem durchzuführen:

     Berechnen von Temperaturmessungen basierend auf reflektiertem Licht von Faser-Bragg-Gittern einer Ablationsvorrichtung während einer Anwendung von Mikrowellenablationsenergie durch die Ablationsvorrichtung auf ein Ziel innerhalb eines Patienten; Extrapolieren der berechneten Temperaturmessungen, um ein Ablationsvolumen in Echtzeit zu simulieren; Bewirken, dass eine Anzeigevorrichtung ein Bild des Ziels anzeigt; und Bewirken, dass die Anzeigevorrichtung eine Progression des Ablationsvolumens auf das Bild des Ziels unter Verwendung einer Simulation eines Ablationswachstums (618) überlagert, die die Progression eines Wachstums der Ablationszone repräsentiert.

13. Verfahren nach Anspruch 12, wobei das Bild des Ziels ein dreidimensionales Rendering eines Teils des Patienten ist, das aus DICOM-Bildern und/oder fluoroskopischen Bildern erzeugt wird.

14. Verfahren nach Anspruch 12 oder Anspruch 13, wobei die Rechenvorrichtung ferner dazu konfiguriert ist, das Verfahren aus Folgendem durchzuführen:

     Berechnen einer gesamten erwarteten Ablationszone; Bewirken, dass die Anzeige die gesamte erwartete Ablationszone auf einem Bild des Ziels innerhalb des Patienten anzeigt; und Bewirken, dass die Anzeige die Progression des Ablationsvolumens innerhalb der Anzeige der gesamten erwarteten Ablationszone anzeigt.

15. Verfahren nach einem der Ansprüche 12 bis 14, wobei die Rechenvorrichtung ferner dazu konfiguriert ist, das Ablationsvolumen basierend auf den extrapolierten Temperaturmessungen, einer Gesamtmenge an angewandter Mikrowellenablationsenergie und einer Dauer einer Energieanwendung zu simulieren.

## Revendications

**1.** Système d'ablation comprenant :

un dispositif d'ablation (130) conçu pour délivrer de l'énergie d'ablation par micro-ondes et comprenant une fibre (300) comportant des réseaux de Bragg ; et

un dispositif informatique (100) comportant un processeur (204) et une mémoire (202) stockant des instructions qui, lors de leur exécution par le processeur, amènent le dispositif informatique à :

recevoir la lumière réfléchie par les réseaux de Bragg (300, 302a, ..., 302n) du dispositif d'ablation (130) ;

calculer des mesures de température en se basant sur la lumière réfléchie pendant l'application de l'énergie d'ablation par micro-ondes par le dispositif d'ablation à une cible dans le corps d'un patient ;

extrapoler les mesures de température calculées pour simuler un volume d'ablation en temps réel ;

amener un dispositif d'affichage (120, 206) à afficher une image (613) de la cible ; et

amener le dispositif d'affichage à superposer une progression du volume d'ablation sur l'image de la cible en utilisant une simulation de croissance d'ablation (618) représentant la progression de la croissance du volume d'ablation.

**2.** Système d'ablation de la revendication 1, le dispositif d'ablation comportant :
une antenne rayonnante (135) conçue pour appliquer de l'énergie d'ablation par micro-ondes à une cible dans le corps d'un patient afin de réaliser l'ablation de la cible, les réseaux de Bragg (302a, ..., 302n) étant formés le long d'une longueur de la fibre (300) et conçus pour réfléchir la lumière provenant de celle-ci.

**3.** Système d'ablation de la revendication 2, dans lequel les réseaux de Bragg sont gravés à l'intérieur de la fibre.

**4.** Système d'ablation de la revendication 2 ou 3, dans lequel le dispositif d'ablation est un cathéter d'ablation par micro-ondes souple (130a) conçu pour être acheminé jusqu'à la cible à travers un réseau luminal d'un patient.

**5.** Système d'ablation de l'une quelconque des revendications 2 à 4, dans lequel le dispositif d'ablation est un dispositif d'ablation par micro-ondes rigide (130b) conçu pour être inséré par voie percutanée à travers un tissu pour accéder à la cible.

**6.** Système d'ablation de l'une quelconque des revendications 2 à 5, dans lequel le dispositif informatique est conçu pour simuler le volume d'ablation en se basant sur les mesures de température extrapolées, une quantité totale d'énergie d'ablation par micro-ondes appliquée, et une durée d'application d'énergie.

**7.** Système de l'une quelconque des revendications 2 à 6, dans lequel le dispositif informatique est conçu pour :

déterminer une géométrie de zone d'ablation prévue (620) devant être formée par le dispositif d'ablation en se basant sur un modèle du dispositif d'ablation ; et

extrapoler les mesures de température en se basant sur la géométrie de zone d'ablation prévue déterminée.

**8.** Système d'ablation de la revendication 7, dans lequel la géométrie de zone d'ablation prévue est de forme sensiblement sphérique, et le dispositif informatique est conçu pour extrapoler les mesures de température en se basant sur la forme sensiblement sphérique.

**9.** Système d'ablation de la revendication 1, dans lequel l'image de la cible est un rendu tridimensionnel d'une partie du corps du patient généré à partir d'images DICOM et/ou d'images fluoroscopiques.

**10.** Système d'ablation de la revendication 1 ou la revendication 9, dans lequel le dispositif informatique est conçu pour :

calculer une zone d'ablation totale prévue ;

amener le dispositif d'affichage à afficher la zone d'ablation totale prévue sur une image de la cible dans le corps du patient ; et

amener le dispositif d'affichage à afficher la progression du volume d'ablation à l'intérieur de l'affichage de la zone d'ablation totale prévue.

**11.** Système d'ablation de l'une quelconque des revendications 1, 9 et 10, dans lequel le dispositif informatique est conçu pour simuler le volume d'ablation en se basant sur les mesures de température extrapolées, une quantité totale d'énergie d'ablation par micro-ondes appliquée, et d'une durée d'application d'énergie.

**12.** Procédé de fonctionnement d'un dispositif informatique dans un système d'ablation, le dispositif informatique comportant un processeur et une mémoire stockant des instructions qui, lors de leur exécution par le processeur, amènent le dispositif informatique à effectuer le procédé suivant :

calcul de mesures de température sur la base de la lumière réfléchie par des réseaux de Bragg sur fibre d'un dispositif d'ablation pendant l'application d'énergie d'ablation par micro-ondes par le dispositif d'ablation à une cible dans le corps d'un patient ;

extrapolation des mesures de température calculées pour simuler un volume d'ablation en temps réel ;

opération consistant à amener un dispositif d'affichage à afficher une image de la cible ; et

opération consistant à amener le dispositif d'affichage à superposer une progression du volume d'ablation sur l'image de la cible, au moyen d'une simulation de croissance d'ablation (618) représentant la progression de la croissance de la zone d'ablation.

13. Procédé de la revendication 12, dans lequel l'image de la cible est un rendu tridimensionnel d'une partie du corps du patient généré à partir d'images DICOM et/ou d'images fluoroscopiques.

14. Procédé de la revendication 12 ou la revendication 13, dans lequel le dispositif informatique est en outre conçu pour effectuer le procédé suivant :

calcul d'une zone d'ablation totale prévue ;

opération consistant à amener le dispositif d'affichage à afficher la zone d'ablation totale prévue sur une image de la cible dans le corps du patient ; et

opération consistant à amener le dispositif d'affichage à afficher la progression du volume d'ablation à l'intérieur de l'affichage de la zone d'ablation totale prévue.

15. Procédé de l'une quelconque des revendications 12 à 14, dans lequel le dispositif informatique est en outre conçu pour simuler le volume d'ablation en se basant sur les mesures de température extrapolées, une quantité totale d'énergie d'ablation par micro-ondes appliquée, et une durée d'application d'énergie.

FIG. 1

EP 4 397 266 B1

**FIG. 2**

**FIG. 3A**

EP 4 397 266 B1

FIG. 3B

FIG. 4

FIG. 5

FIG. 6

700

701
Acquire preoperative CT to identify target(s)

703
Navigate ablation device to identified target

705
Initiate application of microwave ablation
energy

707
Process light reflected from fiber bragg gratings to calculate
temperature (and optionally monitor reflection time)

709
Extrapolate temperature values calculated to generate
ablation volume

711
Display ablation zone volume on display

FIG. 7

**EP 4 397 266 B1**

**Patent documents cited in the description**

- US 2021361353 A